# EUROPEAN PATENT APPLICATION

(11) **EP 2 298 313 A1**
(43) Date of publication of application: **23.03.2011**
(21) Application number: 09746420.0
(22) Date of filing: 03.03.2009
(51) Int. Cl.: A61K 31/5415, A61K 9/06, A61K 9/70, A61K 47/10, A61K 47/22, A61P 1/08, C07D 279/28

(54) **PHARMACEUTICAL COMPOSITION FOR EXTERNAL APPLICATION CONTAINING PROCHLORPERAZINE**

(30) Priority: 15.05.2008 JP 2008128245
(71) Applicant: Nippon Zoki Pharmaceutical Co., Ltd., Osaka-shi Osaka 541-0046 (JP)
(72) Inventor: OBATA, Yasuko, Kawasaki-shi Kanagawa 210-0834 (JP); OTAKE, Yuki, Tokyo 142-0051 (JP); TAKAYAMA, Kozo, Tokyo 145-0067 (JP)
(74) Representative: HOFFMANN EITLE
(86) International application number: PCT/JP2009/053988
(87) International publication number: WO 2009/139213

(57) **Abstract**

Disclosed is a novel pharmaceutical composition for external application for increasing the permeation of prochlorperazine or a pharmaceutically acceptable salt thereof through the skin to allow prochlorperazine or the pharmaceutically acceptable salt thereof to exhibit its excellent pharmacological activity. Specifically disclosed is a novel pharmaceutical composition for external application which contains prochlorperazine or a pharmaceutically acceptable salt thereof as an active ingredient and further contains menthol. The composition contains, as an active ingredient, prochlorperazine which has been widely used clinically as the first-line drug of a therapeutic agent for nausea or vomiting before or after a surgery or the like, and is extremely highly useful as a transdermally absorbable antiemetic agent that has excellent efficacy and car be used safely over a long period.

## Description

### Technical Field

The present invention relates to a pharmaceutical composition for external application of a percutaneous absorption type containing prochlorperazine or a pharmaceutically acceptable salt thereof as an effective ingredient.

### Background Art

Prochlorperazine [2-chloro-10-[3-(4-methyl-piperazin-1-yl)prdpyl]-10H-phenothiazine] is classified as an antipsychotic drug of a phenothiazine type and, in Japan, prochlorperazine maleate tablets and prochlorperazine mesylate injection have been put into the market in 1957 and 1959, respectively, where schizophrenia (for tablets only) and nausea/vomiting before and after the operation, etc. are the indications. Prochlorperazine has a dopamine D₂ receptor antagonistic action and suppresses the vomiting by acting on chemoreceptor trigger zone (CTZ) in the area postrema of medulla. Its antiemetic action has been said to be four to five times stronger as compared with chlorpromazine which is also a phenothiazine-type antipsychotic drug and has been widely used in clinical fields as the first choice for the treatment of nausea and vomiting before and after the operation, etc.

Generally, a method where a pharmaceutical agent is administered by means of percutaneous absorption is able to solve various problems such as that the administration frequency is reduced by sustaining of effective concentrations in blood, that the adverse effect is avoided by lowering of the maximum blood level, that the pain upon injection and infusion is removed, that the application to treatment at home is possible, that the hepatic first-pass effect upon oral administration is avoided or that the compliance and the quality of life of patients (QOL) are improved as compared with the administration of injections and peroral agents. However, some types of the pharmaceutical agents are hardly absorbed through the skin and there are also many cases where it is difficult to make into the preparations for external application.

Particularly in the case of antiemetic drugs such as prochlorperazine, they are inevitably administered to patients who need the treatment for nausea (unusual sense of unpleasant and vomiting feel taking in the throat to the upper abdomen which often happens prior to vomiting) and vomiting (a phenomenon that the content in the stomach is discharged from the mouth via esophagus and oral cavity as a result of sudden shrinking of diaphragm and abdominal muscle) and it is often that oral administration to the patients as such is difficult. Accordingly, there has been a demand in the practical clinical field for an antiemetic agent of a percutaneous type which is able to be easily and conveniently administered even to the patients exhibiting the symptoms of nausea and vomiting.

With regard to prochlorperazine however, the preparations which have now been sold as the products in Japan are tablets and injections only while there is no example of the preparation for external application and there has been almost no report for the percutaneously absorptive preparation of prochlorperazine. Only in the international publication WO 01/35883 (Patent Document 1), prochlorperazine is disclosed in its Example V-21 as one of antiemetic agents to be added to percutaneously absorptive preparations. This document discloses an invention for percutaneously absorptive preparations of aconitine (a kind of alkaloids) and, as one of the pharmaceuticals which are able to be made co-present in aconitine, antiemetic agents are exemplified. Among them, prochlorperazine is merely disclosed as one of the antiemetic agents and no specific composition is disclosed.

Patent Document 1: International Publication WO 01/35883

### Disclosure of the Invention

### Problems that the Invention is to Solve

An object of the present invention is to provide a novel pharmaceutical composition for promoting the percutaneous permeation of prochlorperazine or a pharmaceutical acceptable salt thereof which is an effective ingredient so as to achieve an excellent pharmacological effect.

### Means for Solving the Problems

Under the current state where there has been almost no report for an externally applicable prochlorperazine preparation, the present inventors have carried out intensive studies for the percutaneous absorbability of prochlorperazine which is hardly absorbed through the skin and investigated for its preparation in order to achieve an excellent pharmacological effect as an externally applicable agent. As a result, they have found that, when menthol is compounded with prochlorperazine, the percutaneous absorbability of prochlorperazine is promoted. The present inventors also found the components by which the percutaneous absorbability of prochlorperazine is further improved when they are added in the manufacture of hydrogel and adhesive matrix which are particularly able to be applied to ointment and plaster, respectively.

### Advantages of the Invention

The pharmaceutical composition for external application according to the present invention contains prochlorperazine which has been widely used in clinical fields as the first choice for the treating agent for nausea/vomiting before and after the operation, etc. as an effective ingredient and the resulting composition is highly useful being excellent in its effect, being able to be safely used for a long period of time, having high convenience in practical use and being applicable to antiemetic agent, etc. of a percutaneously absorptive type.

### Brief Description of Drawings

Fig. 1 is a graph which shows the result of accumulated skin-permeating amount with elapse of time of prochlorperazine (hereinafter, it will be sometimes referred to as "PCP") of the formulation 1 in an *in vitro* skin permeation test.
Fig. 2 is the result of an *in vitro* skin permeation test showing the skin permeating amount of PCP of the formulations 3, 4, 5, 6 and 1 where the adding amount of PCP is varied.
Fig. 3 is the result of an *in vitro* skin permeation test showing the skin permeating amount of PCP of the formulations 1 and comparative formulations 3 and 4 (C3 and C4) where additives are 1-menthol, d-limonene and Tween 80, respectively.
Fig. 4 is the result of an *in vitro* skin permeation test showing the skin permeating amount of PCP of the comparative formulations 1 and 2 (C1 and C2) and the formulations 7 and 1 where the adding amount of 1-menthol is varied.
Fig. 5 is the result of an *in vitro* skin permeation test showing the skin permeating amount of PCP of the formulations 8 and 1 where the adding amount of N-methyl-2-pyrrolidone is varied.
Fig. 6 is the result of an *in vitro* skin permeation test showing the skin permeating amount of PCP of the formulations 1 and 2 where isopropanol and ethanol, respectively, are used as a lower alcohol.
Fig. 7 is a graph which shows the result of an *in vitro* skin permeation test for accumulated skin-permeating amount with elapse of time of PCP of the plasters where the type of the adhesive is varied.
Fig. 8 is a graph which shows the result of an *in vitro* skin permeation test for skin permeating amount of PCP of the plasters where the type of the adhesive is varied.
Fig. 9 is the result of an *in vivo* anti-apomorphine test showing the numbers of stereotyped behaviors induced by apomorphine after 4 hours from the application of the hydrogel in the group being applied with the hydrogel of the formulation 1 and in the control group.
Fig. 10 is the result of an *in vivo* anti-apomorphine test showing the numbers of stereotyped behaviors induced by apomorphine after 8 hours from the application of the hydrogel in the group being applied with the hydrogel of the formulation 1 and in the control group.

### Best Mode for Carrying Out the Invention

The present invention relates to a novel pharmaceutical composition for external application in which prochlorperazine or a pharmaceutically acceptable salt thereof is an effective ingredient and menthol is made to contain therein. Particularly in the composition of the present invention in a form of hydrogel, it is preferred that the composition contains lower alcohol and/or pyrrolidone compound in addition to menthol. Further, in the case of the composition of the present invention in form of an adhesive matrix, it is preferred that the composition contains polyvinylpyrrolidone, glycerol fatty acid ester and/or fatty acid ester in addition to menthol.

Prochlorperazine which is the effective ingredient of the pharmaceutical composition for external application according to the present invention includes its salt and there is no particular limitation for the salt so far as it is a pharmaceutically acceptable acid addition salt. Examples thereof include an inorganic acid salt such as hydrochloride, sulfate, nitrate, phosphate, hydrofluoride or hydrobromide and an organic acid salt such as acetate, tartrate, lactate, citrate, fumarate, maleate, mesylate, succinate, methanesulfonate, benzenesulfonate, toluenesulfonate, naphthalenesufonate or camphorsulfonate. Preferred examples include prochlorperazine maleate and mesylate which have been commercially available and widely used in the clinical fields. Hydrate and solvate of prochlorperazine are also covered in the prochlorperazine which is an effective ingredient of the present invention.

Compounding amount of prochlorperazine or a pharmaceutically acceptable salt thereof contained in the pharmaceutical composition for external application according to the present invention as an effective ingredient is able to be appropriately selected depending upon the dosage form, etc. which will be mentioned later and, in the case of a hydrogel form, it may be made, for example, 0.1 to 5% by weight, preferably 0.2 to 3% by weight and, more preferably, 0.3 to 1% by weight to the total weight of the composition while, in the case of an adhesive matrix form, it may be made, for example, 0.5 to 30% by weight and, preferably, 1 to 20% by weight to the total weight of the composition.

Menthol used in the pharmaceutical composition for external application according to the present invention includes 1-menthol and dl-menthol which are optical isomers thereof. Peppermint oil and peppermint water where menthol is a main component are also covered by menthol of the present invention. Compounding amount of menthol used is able to be appropriately selected depending upon the dosage form, etc. which will be mentioned later and, in the case of a hydrogel form, it may be made, for example, 0.75 to 10% by weight and, preferably, 1 to 5% by weight to the total weight of the composition while, in the case of an adhesive matrix form, it may be made, for example, 0.5 to 20% by weight and, preferably, 1 to 15% by weight to the total weight of the composition.

Examples of the lower alcohol which is used in the pharmaceutical composition for external application according to the present invention include methanol, ethanol, propanol, isopropanol, butanol, isobutanol and 2-butanol. Compounding amount of the lower alcohol used is able to be appropriately selected depending upon the dosage form, etc. which will be mentioned later and, in the case of a hydrogel form, it may be made, for example, 5 to 40% by weight and, preferably, 10 to 30% by weight to the total weight of the composition.

Examples of the pyrrolidone compound which is used in the pharmaceutical composition for external application according to the present invention include 2-pyrrolidone, N-methyl-2-pyrrolidone, 5-methyl-2-pyrrolidone, 1,5-dimethyl-2-pyrrolidone, 1-ethyl-2-pyrrolidone and 1-dodecyl-2-pyrrolidone2-pyrrolidone N-Methyl-2-pyrrolidone is particularly preferred. Compounding amount of the pyrrolidone compound used is able to be appropriately selected depending upon the dosage form, etc. which will be mentioned later and, in the case of a hydrogel form, it may be made, for example, 0.1 to 40% by weight and, preferably, 0.5 to 25% by weight to the total weight of the composition. Polyvinylpyrrolidone used in an adhesive matrix is a macromolecular compound where N-vinyl-2-pyrrolidone is polymerized and it may be made, for example, 1 to 30% by weight and, preferably, 2 to 20% by weight to the total weight of the composition.

As to the adhesive base used in the pharmaceutical composition for external application according to the present invention, a silicone-type adhesive is suitable. As to the silicone-type adhesive, that where polyorganosiloxane such as polydimethylsiloxane, polymethylphenylsiloxane or polydiphenylsiloxane is a main component may be exemplified and, in the commercially available silicone-type adhesive, a BIO-PSA (trade name) series manufactured by Dow Corning is able to be used. Compounding amount of the silicone-type adhesive used is able to be appropriately selected depending upon the dosage form, etc. which will be mentioned later and, in the case of an adhesive matrix form, it may be made, for example, 20 to 96% by weight and, preferably, 30 to 92% by weight to the total weight of the composition.

As to the glycerol fatty acid ester used in the pharmaceutical composition for external application, a medium-chain (having 5 to 25 carbons) fatty acid glycerol ester may be exemplified. More specific examples include glycerol caprylate, glycerol caprate, glycerol laurate, glycerol palmitate, glycerol monooleate, glycerol stearate, glycerol dicaprylate, glycerol tricaprylate, glycerol di-tricaplyrate and sorbitan caprate. Preferred examples include glycerol monocaprylate, glycerol monocaprate and glycerol monolaurate and, among them, glycerol monooleate is particularly preferred. Each of those glycerol fatty acid esters may be used solely or two or more thereof may be mixed and used. Compounding amount of the glycerol fatty acid ester used is able to be appropriately selected depending upon the dosage form, etc. which will be mentioned later and, in the case of an adhesive matrix form, it may be made, for example, 1 to 30% by weight and, preferably, 2 to 20% by weight to the total weight of the composition.

As to the fatty acid ester used in the pharmaceutical composition for external application according to the present invention, fatty acid ester comprising fatty acid where carbon numbers are 6 to 22 and alcohol where carbon number(s) is/are 1 to 12 may be exemplified. Examples of the fatty acid having 6 to 22 carbons include a monocarboxylic acid such as caproic acid, enanthic acid, caprylic acid, capric acid, undecylenic acid, lauric acid, myristic acid, palmitic acid, margaric acid, stearic acid, oleic acid or linoleic acid; and a dicarboxylic acid such as adipic acid or sebacic acid. Examples of the alcohol having 1 to 12 carbon(s) include methanol, ethanol, propanol, isopropanol, butanol, tert-butanol, hexanol and octanol. Accordingly, examples of the fatty acid ester include diisopropyl adipate, diethyl sebacate, isopropyl myristate, isopropyl palmitate, isopropyl stearate, butyl stearate, octyldodecyl myristate, butyl myristate, hexyl laurate, octyl palmitate and ethyl oleate. Isopropyl myristate is particularly preferred. Each of those fatty acid esters may be used solely or two or more thereof may be mixed and used. Compounding amount of the fatty acid ester is able to be appropriately selected depending upon the dosage form, etc. which will be mentioned later and, in the case of an adhesive matrix, it may be made, for example, 1 to 30% by weight and, preferably, 2 to 20% by weight to the total weight of the composition.

There is no particular limitation for the specific dosage form of the composition for external application of the present invention and, although various kinds of percutaneous application agents which have been usually available in the market under various names may be prepared, the composition is suitable for the production of ointment or plaster. In the present specification and claims, the term "ointment" stands for an externally applying preparation to be applied to the skin which is usually prepared in a wholly uniform semisolid form with an appropriate consistency and includes the preparations called cream preparation, gel preparation, lotion preparation, etc. Similarly, the term "plaster" stands for an externally applying preparation usually spread on cloth or plastic film or sealed therein and used by adhering to the skin and includes the preparations called tape preparation, patch preparation, etc. In making into the preparation, it is able to be manufactured by appropriately using additives and base materials suitable for each of the dosage forms and subjected to common methods disclosed, for example, in the General Rules for Manufacturing the Preparations mentioned in the Japanese Pharmacopoeia. In the formulation, other pharmaceutically active ingredient may be also used together with prochlorperazine to give a compounded preparation.

Examples of the preferred dosage form of the composition for external application according to the present invention include the hydrogel where sticky feel is little and application to ointment is possible and the adhesive matrix which is able to be applied to a plaster of a matrix type having excellent adhesion and using feel to the skin. In preparing the hydrogel, it is possible to use a commonly conducted method where a thickener such as hydroxyethyl cellulose, hydroxypropyl cellulose, hydroxypropyl methyl cellulose or carboxyvinyl polymer is used followed by swelling with water to give a base and, in order to uniformly mix prochlorperazine which is a main ingredient and the above absorption promoter, etc. in a preparation, a suitable solvent such as isopropanol or ethanol is used whereupon an ointment is able to be prepared. In preparing an adhesive matrix, it is preferred to use a silicone-type adhesive as an adhesive base and to use a common method where the compounding components such as an absorption promoter are uniformly dissolved in a solvent which is in the same type as the solvent contained in said adhesive and mixed with a adhesive to make homogeneous. When this adhesive matrix is applied on a support such as polyethylene terephthalate followed by drying, a plaster is able to be prepared.

### Examples

The present invention will now be more specifically illustrated by way of the following Examples and Comparative Examples although the present invention is not limited thereto.

### Example 1: Preparation of hydrogel

Hydrogel of the formulation of the present invention as shown in Table 1 and that of the comparative control formulation as shown in Table 2 were prepared according to the following methods. Water was added to hydroxyethyl cellulose, hydroxypropyl cellulose and PCP (all of the PCP used in Examples was a maleate and, even in that case, it is expressed as PCP for the sake of convenience) and allowed to stand for one night whereupon the base was swollen. On the other hand, other components in the formulations shown in Tables 1 and 2 (isopropanol, ethanol, 1-menthol, d-limonene, Tween 80 and N-methyl-2-pyrrolidone) were mixed therewith upon necessity and gradually added to the base to prepare a homogeneous hydrogel. Amount of water was adjusted as the compounding amounts of PCT and other component increase or decrease so that the total amount was made 100% by weight.

**Table 1**

| Compounding Components (% by weight) | Formulation 1 | Formulation 2 | Formulation 3 | Formulation 4 | Formulation 5 | Formulation 6 | Formulation 7 | Formulation 8 |
|---|---|---|---|---|---|---|---|---|
| Prochlorperazine (PCP) | 1 | 1 | 0.3 | 0.5 | 0.6 | 0.75 | 1 | 1 |
| Isopropanol | 20 | - | 20 | 20 | 20 | 20 | 20 | 20 |
| Methanol | - | 20 | - | - | - | - | - | - |
| 1-Menthol | 2 | 2 | 2 | 2 | 2 | 2 | 1 | 2 |
| d-Limanene | - | - | - | - | - | - | - | - |
| Tween 80 | - | - | - | - | - | - | - | - |
| Hydroxyethyl cellulose | 1 | 1 | 1 | 1 | 1 | 1 | 1 | 1 |
| Hydroxypropyl cellulose, | 1 | 1 | 1 | 1 | 1 | 1 | 1 | 1 |
| N-Methyl-2-pyrrolidone | 10 | 10 | 10 | 10 | 10 | 10 | 10 | - |

**Table 2**

| Compounding Components (% by weight) | Comparative Formulation 1 | Comparative Formulation 2 | Comparative Formulation 3 | Comparative Formulation 4 |
|---|---|---|---|---|
| Prochlorperazine (PCP) | 1 | 1 | 1 | 1 |
| Isopropanol | 20 | 20 | 20 | 20 |
| Ethanol | - | - | - | - |
| I-Menthol | - | 0.5 | - | - |
| d-Limonene | - | - | 2 | - |
| Tween 80 | - | - | - | 2 |
| Hydroxyethyl cellulose | 1 | 1 | 1 | 1 |
| Hydroxypropyl cellulose | 1 | 1 | 1 | 1 |
| N-Methyl-2-pyrrolidone | 10 | 10 | 10 | 10 |

### Example 2: In vitro Skin Permeation Test

The skin excised from abdomen of male hairless mouse (seven weeks age) was cut out in full thickness and mounted on a Franz type diffusion cell (effective diffusing area: 2.0 cm²; receiver cell volume: 16.0 mL). The hydrogel (1 mL) prepared in the above Example 1 was applied into a donor cell and the receiver cell was filled with a phosphate-buffered physiological saline (to which 4% BriJ98 (trade name) was added) of pH 7.4. Each 0.2 mL of the solution was collected every hour from the receiver cell (immediately after the collection, the same amount of the phosphate-buffered physiological saline to which 4% BriJ98 (trade name) was supplemented) and the PCP was quantified by a high performance liquid chromatography (HPLC). Thus, 0.02 mL of methanol in which amitriptyline was dissolved (0.25 µg/mL) as an internal standard, then 0.02 mL of IN sodium hydroxide solution and 1.0 mL of chloroform were added to 0.1 mL of the collected sample, the mixture was mixed for one minute and centrifuged (300 × g for 15 minutes at 24°C) and the organic phase was separated. This was heated at 39°C whereby the organic phase was completely evaporated in a nitrogen stream, 0.1 mL of methanol was added to the residue and the resulting mixture was used as a sample for the measurement by infusing into HPLC after stirring. Measurement of the PCP amount by HPLC was carried out under the condition where the mobile phase was a mixture of methanol and ammonium acetate buffer (0.005M, pH 5.25) in a ratio of 85:15; the column was Inertsil (trade name) CN-3 (250 mm × 4.6 mm); the wave length was 254 mm; the flow rate was 1.6 mL/minute; and the column temperature wads 24°C ± 1°C. Each test was conducted for five times and the mean value ± standard deviation were determined (that was also the same in the following measurement).

### (1) Accumulated permeated amount of PCP through the skin

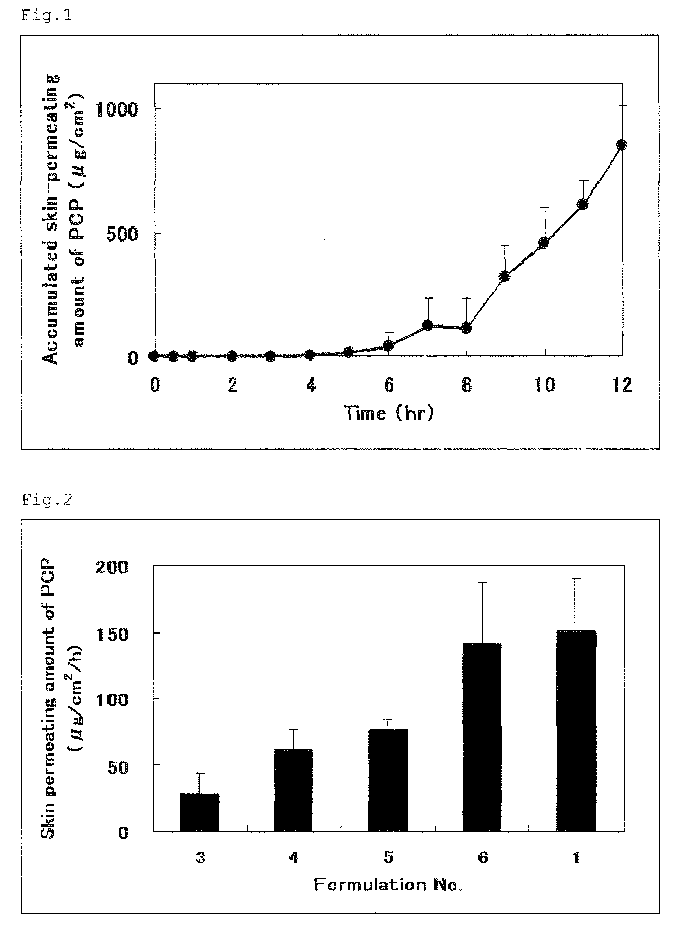

Fig. 1 shows a graph of an accumulated permeated amount vs. time curve showing the accumulated permeated amount through the skin of PCP per unit area of the formulation 1 measured with elapse of in accordance with the above. As will be apparent from this graph, in the composition of the present invention containing 1-menthol, skin permeation of the PCP through the skin was promoted with elapse of time.

Then, results of the tests of the following (2) to (5) are shown using the skin permeated amount of PCP determined from the above measured values (Flux: µg/h/cm²) [this is inclination of the regression straight line resulted by a linear regression of plots of the accumulated permeation amount vs. time curve].

### (2) Influence of added amount of PCP on the skin permeated amount of PCP

Fig. 2 shows the skin permeating amounts of PCP in the formulations 3, 4, 5, 6 and 1 where only adding amount of PCP was varied as 0.3% by weight, 0.5% by weight, 0.6% by weight, 0.75% by weight and 1% by weight, respectively, while other compounding components were the same as shown in Table 1. From the result, it is noted that the skin permeating amount increases depending upon the added amount of PCP.

### (3) Influence of additives on the skin permeated amount of PCP

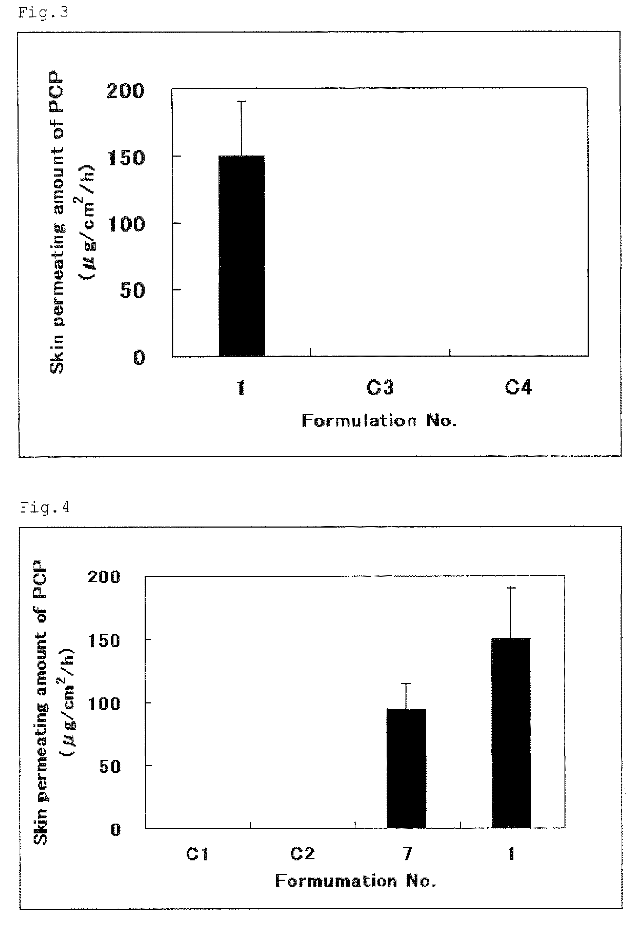

Fig. 3 shows the skin permeating amount of PCP in the formulation 1 and the comparative formulations 3 and 4 where the additives were 1-menthol, d-limonene and Tween 80, respectively while other compounding components were the same. The results show that, when 1-menthol was used as an additive, significantly high skin permeation was noted whereby it was confirmed to be favorable as an absorption promoter. Although d-limonene is a monocyclic monoterpene like 1-menthol and is a compound having the similar structure, PCP rarely showed the skin permeation when d-limonene was added. Further, although Tween 80 is a surfactant having emulsifying and dispersing actions besides the permeating action due to lowering of surface tension of aqueous solution, PCP also rarely showed the skin permeation when Tween 80 was added.

### (4) Influence of added amount of additives on the skin permeated amount of PCP

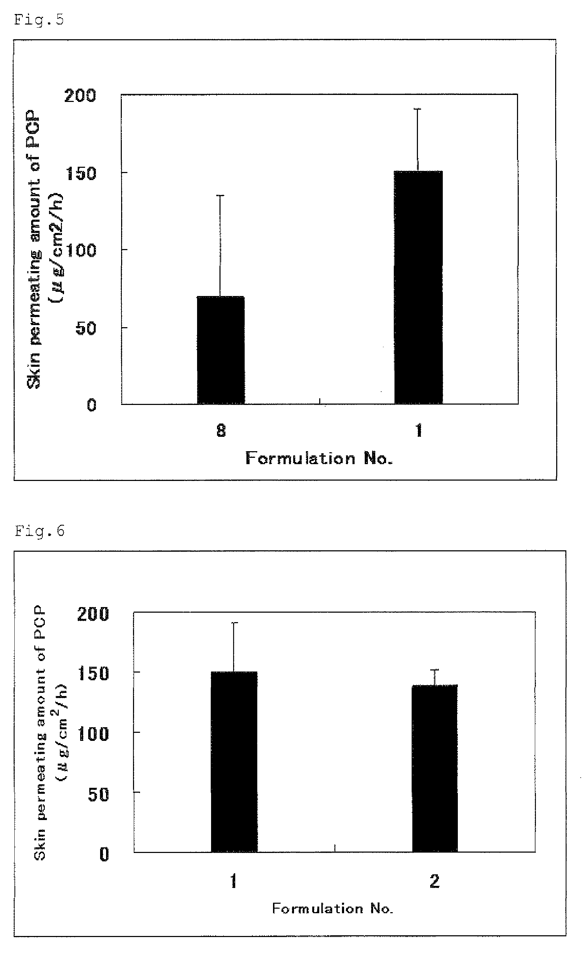

Fig. 4 shows the skin permeated amount of PCP in the comparative formulations 1, 2 and 7 and the formulation 1 where only added amount of 1-menthol was made 0% by weight, 0.5% by weight, 1% by weight and 2% by weight, respectively, while other compound components were made same. Fig. 5 shows the skin permeated amount of PCP in the formulations 8 and 1 where only added amount of N-methyl-2-pyrrolidone was made 0% by weight and 10% by weight, respectively. From those results, with regard to 1-menthol, significant skin permeability was noted when its added amount of 1% by weight or more. With regard to N-methyl-2-pyrrolidone, significant skin permeability was noted when the added amount was 10% by weight or more and, although some skin permeability was available even when it was not added, some imbalance in the skin permeability was noted whereby it was noted that addition was more preferred.

### (5) Influence of lower alcohol on the skin permeated amount of PCP

Fig. 6 shows the skin permeated amount of PCP in the formulations 1 and 2 where the lower alcohol was isopropanol and ethanol, respectively, while other compounding components were same. From the results, it was noted that there was almost no difference between the two in terms of the skin permeability and that various lower alcohols were able to be appropriately used.

### Example 3: Preparation of plaster using adhesive matrix

Four kinds of PCP-containing pressure-sensitive adhesive plasters where the adhesives were different were prepared by the following method using the adhesives shown in Table 3.
PCP, 1-menthol, polyvinylpyrrolidone, glycerol monooleate and isopropyl myristate of 20%, 20%, 15%, 20% and 10% by weight, respectively, to the weight of each adhesive were dissolved in a solvent (in an amount of 1.5-timers of the adhesive) contained in each adhesive, mixed for 1 minute and subjected to an ultrasonic treatment for 10 minutes to give uniform solutions. Each of them was added to the corresponding adhesive and stirred for one night using a stirrer whereupon the drug and the additives were dispersed. An adhesive matrix which was a mixed solution or a dispersion of adhesive, PCP and additives made into uniform was placed on a support (polyethylene terephthalate film of 38 µm) fixed on a glass plate and applied in a thickness of 300 µm. It was air-dried for two days in a draft chamber to remove the solvent whereupon a plaster was prepared. Final concentrations of the compounded components contained in each adhesive matrix for each adhesive are as shown in Table 4.

**Table 3**

| Adhesive | Composition or Product Name | Solid (%) | Solvent |
|---|---|---|---|
| | . 2-Ethylhexyl acrylate | | |
| | . Butyl acrylate | | |
| Acrylic adhesive | . Methacrylic acid | 50 | Toluene |
| | . N-Vinylpyrrolidone | | |
| | . Dimethylacrylamide | | |
| | . Block copolymer of styrene/isoprene/styrene | | |
| Rubber adhesive | . Rosin ester | 66 | Toluene |
| | . Liquid paraffin | | |
| Silicone adhesive A | Methylphenyl silicone | 55 | Toluene Xylene |
| Silicone adhesive B | BIO-PSA®74502 (Dow Corning) | 60 | Ethyl acetate |

**Table 4**

| Compounded Components (% by weight) | Acrylic Adhesive | Rubber Adhesive | Silicone Adhesive A | Silicone Adhesive B |
|---|---|---|---|---|
| Prochlorperazine (PCP) | 15 | 13 | 14 | 14 |
| I-Menthol | 15 | 13 | 14 | 14 |
| Polyvinylpyrrolidone | 11 | 10 | 11 | 10 |
| Glycerol monooleate | 15 | 13 | 14 | 14 |
| Isopropyl myristate | 7 | 7 | 7 | 7 |
| Adhesive | 37 | 44 | 40 | 41 |

### Example 4: In vitro skin permeability test

Laboskin collected from hairless mouse was placed in a Franz type diffusion cell which was the same as that in Example 2 and, immediately thereafter, each of the plasters prepared in Example 3 was applied by lightly pushing to the horny layer side of the laboskin and then a receiver cell was installed. In the receiver cell side, 16 mL of a phosphate-buffered physiological saline of pH 7.4 to which 4% of nonionic surfactant BriJ98 (trade name) was added was placed and the cell was heated on a warm bath of 37°C. Each 0.2 mL of the solution was collected every hour from the receiver cell and PCP was quantified by HPLC according to the similar manner as in Example 2.

### Influence of adhesive base on the accumulated skin permeation amount of PCP

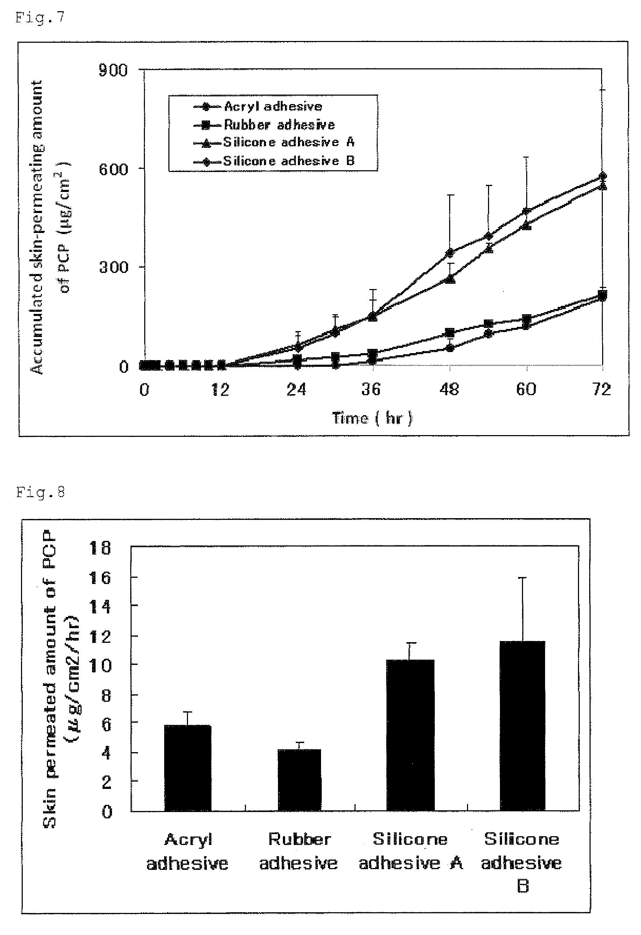

Fig. 7 shows a graph of accumulated permeation amount vs. time curve showing the accumulated permeation amount of PCP per unit area of each plaster measured with elapse of time. As will be apparent from this result, in a plaster prepared using an adhesive matrix comprising the composition of the present invention, an excellent accumulated skin permeability of PCP was noted when a silicone adhesive was used as an adhesive base as compared with the case where acrylic adhesive or rubber adhesive was used.

### (2) Influence of adhesive base on the skin permeating amount of PCP

Fig. 8 shows a skin permeating amount (Flux: g/h/cm²) of PCP determined from the above measured values for each of the adhesives. The same as in the result of the above Fig. 7, an excellent skin permeation amount (high flux value) was achieved when a silicone adhesive was used as an adhesive base in a plaster prepared by using an adhesive matrix comprising the composition of the present invention.

### Example 5: In vivo anti-apomorphine test

In order to confirm that PCP achieves its pharmacological effect by being delivered into living body through the skin, a suppressive effect of PCP to the stereotyped behavior of mouse induced by apomorphine was evaluated by means of observation of the behavior. The test was carried out by referring to a method by Takayasu, et al. (Drug Delivery System, original article, volume 11, no. 3, pages 197 to 203, May 1996). Each of the tests was conducted for five times to determine mean value ± standard error.

### (1) First day of the test

Hair on the back of male mice (seven weeks age) of ddY strain was shaved using a clipper for animals under anesthetizing with pentobarbital (50mg/kg) within such an area that was able to be equipped with a glass cell of an effective area of 2 cm². After the shaving, feed and water were placed in a cage for one night.

### (2) Second day of the test

The mice where the back was shaved were placed in tubs for observation by dividing into a group to which a hydrogel of the formulation 1 was applied and a control group and then spontaneous movement and standing-up behavior were observed for five minutes. As to the spontaneous movement, the case where the body of a mouse completely went over the line drawn on the tub was counted as one time and, as to the standing-up behavior, the case where the both forelimbs were completely separated from the floor was counted as one time. After that, a glass cell was adhered, using an adhesive for living body (trade name: Aron Alpha), to the shaved back of the mice in each group under anesthetization with pentobarbital (50 mg/kg). In the adhered glass cell, 1 mL of PCP hydrogel prepared in Example 1 was placed for the group to which the hydrogel of the formulation 1 was applied while, for the control group, 1 mL of hydrogel prepared by removing PCP from the formulation 1 was placed. After the cell was covered with a plastic cover, it was covered with a film made of paraffin.

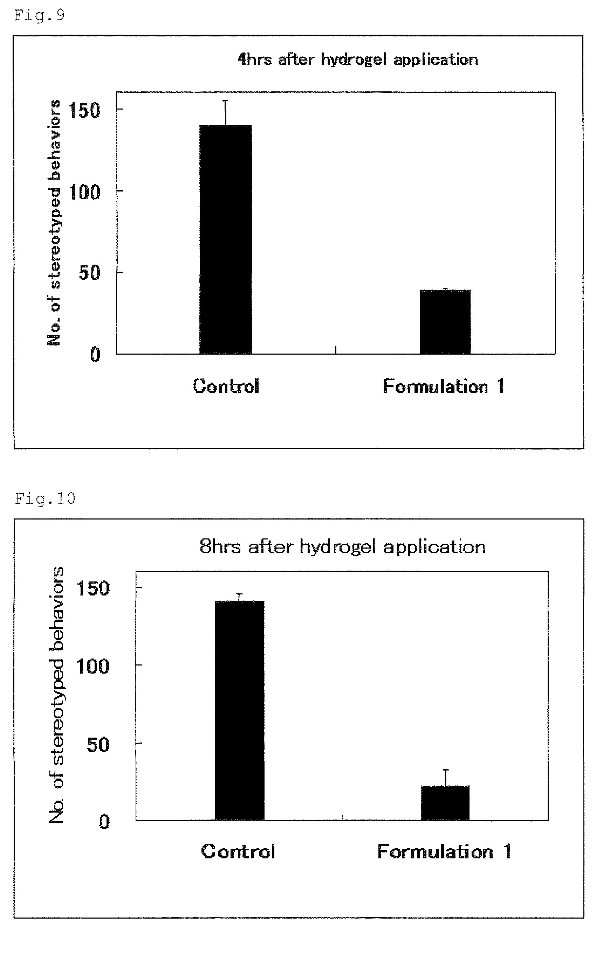

After 4 hours and 8 hours from the applications of the hydrogel, an apomorphine hydrochloride solution (0.1 mg/mL) dissolved in a physiological saline was subcutaneously administered (1 mg/kg) to the mice of each group, the mice were then placed in an open field made of plastics and their spontaneous movements and standing-up behaviors for five minutes were measured. The result after 4 hours from the hydrogel application is shown in Fig. 9 and the result after 8 hours from the hydrogel application is shown in Fig. 10. When the group to which the hydrogel of the formulation 1 was applied and the control group was compared, it is apparent from Figs. 9 and 10 that, in the hydrogel application group, PCP was absorbed through the skin and the stereotyped behavior induced by apomorphine was suppressed. It was noted that, even after 4 hours from the application of the hydrogel of the formulation 1, an excellent pharmacological effect was achieved and, after 8 hours, far better pharmacological effect was resulted.

### Industrial Applicability

As will be apparent from the above results of the pharmacological tests, it is now possible to promote the percutaneous absorption of prochlorperazine and to achieve a significant pharmacological effect when menthol is made to contain. Further, in the hydrogel form of the pharmaceutical composition of the present invention, the skin permeability of prochlorperazine is able to be improved in a more stable manner by addition of a lower alcohol such as isopropanol or ethanol or by addition of N-methyl-2-pyrrolidone. When the hydrogel as such is utilized, manufacture of ointment, etc. having an excellent percutaneous absorption is possible. Furthermore, in the adhesive matrix form of the pharmaceutical composition of the present invention, it is also possible to improve the percutaneous permeability of prochlorperazine when a silicone adhesive is used as an adhesive base and polyvinylpyrrolidone, glycerol fatty acid ester and/or fatty acid ester are/is further added as additive (s) in addition to menthol. When the adhesive matrix as such is utilized, it is now possible to manufacture a plaster showing an excellent percutaneous absorption. The pharmaceutical composition of the present invention contains prochlorperazine which has been widely used in clinical fields as the first choice, etc. for the treating agent for nausea and vomiting before and after the operation, etc. and the preparation thereof for external application is very highly useful as an antiemetic agent, etc. being excellent in percutaneous absorption and able to be used for a long period.

## Claims

1. A pharmaceutical composition for external application where prochlorperazine or a pharmaceutically acceptable salt thereof is an effective ingredient and menthol is made to contain therein.

2. The pharmaceutical composition for external application according to claim 1, wherein the effective ingredient is prochlorperazine maleate.

3. The pharmaceutical composition for external application according to claim 1, wherein the effective ingredient is prochlorperazine mesylate.

4. The pharmaceutical composition for external application according to any of claims 1 to 3, wherein menthol is 1-menthol.

5. The pharmaceutical composition for external application according to any of claims 1 to 4, wherein the composition further contains a lower alcohol.

6. The pharmaceutical composition for external application according to claim 5, wherein the lower alcohol is isopropanol.

7. The pharmaceutical composition for external application according to claim 5, wherein the lower alcohol is ethanol.

8. The pharmaceutical composition for external application according to any of claims 1 to 7, wherein the composition further contains a pyrrolidone compound.

9. The pharmaceutical composition for external application according to claim 8, wherein the pyrrolidone compound is N-methyl-2-pyrrolidone.

10. The pharmaceutical composition for external application according to any of claims 1 to 9, wherein the composition is in a hydrogel form.

11. A pharmaceutical composition for external application where the prochlorperazine or a pharmaceutically acceptable salt thereof is an effective ingredient and said composition contains menthol, lower alcohol and pyrrolidone compound and is in a hydrogel form.

12. A pharmaceutical composition for external application where the prochlorperazine or a pharmaceutically acceptable salt thereof is an effective ingredient and said composition contains 1-menthol, ethanol or isopropanol and N-methyl-2-pyrrolidone and is in a hydrogel form.

13. An ointment which is prepared using the pharmaceutical composition for external application mentioned in any of claims 1 to 12.

14. The pharmaceutical composition for external application according to any of claims 1 to 4, wherein the composition further contains polyvinylpyrrolidone, glycerol fatty acid ester and/or fatty acid ester and in a form of adhesive matrix where a silicone adhesive is used.

15. The pharmaceutical composition for external application according to claim 14, wherein the glycerol fatty acid ester is glycerol monooleate.

16. The pharmaceutical composition for external application according to claim 14 or 15, wherein the fatty acid ester is isopropyl myristate.

17. A pharmaceutical composition for external application where the prochlorperazine or a pharmaceutically acceptable salt thereof is an effective ingredient and said composition contains 1-menthol, polyvinylpyrrolidone, glycerol monooleate and/or isopropyl myristate and is in an adhesive matrix form using a silicone adhesive.

18. A plaster which is prepared using the pharmaceutical composition for external application mentioned in any of claims 14 to 17.

19. An antiemetic agent which is prepared using the pharmaceutical composition for external application mentioned in any of claims 1 to 12, 14 and 17.

20. The ointment of claim 13 or the plaster of claim 19 which is an antiemetic agent.
